# EUROPEAN PATENT APPLICATION

(11) **EP 4 751 570 A1**
(43) Date of publication of application: **03.06.2026**
(21) Application number: 24845692.3
(22) Date of filing: 26.07.2024
(51) Int. Cl.: A01N 37/44, A01G 7/06, A01H 3/04, A01P 21/00

(54) **PLANT ROOT SYSTEM MORPHOLOGY CONTROL AGENT**

(30) Priority: 27.07.2023 JP 2023122555
(71) Applicant: RIKEN, Wako-shi, Saitama 351-0198 (JP)
(72) Inventor: TABETA Hiromitsu, Wako-shi, Saitama 351-0198 (JP); HIRAI Masami, Wako-shi, Saitama 351-0198 (JP)
(74) Representative: Predazzi, Valentina
(86) International application number: PCT/JP2024/026788
(87) International publication number: WO 2025/023318

(57) **Abstract**

Providing an accessible and effective means of controlling root system morphology of plants.

The present invention provides a root system morphology regulator of plants consisting of 2-aminopimelic acid or a salt or ester thereof.

## Description

### Technical Field

The present invention relates to a root system morphology regulator of plants consisting of 2-aminopimelic acid or a salt or ester thereof. The present invention also relates to a method for controlling root system morphology of plants and a plant cultivation method using 2-aminopimelic acid or a salt or ester thereof.

### Background Art

In plants, roots are essential organs for absorbing water, inorganic salts and other nutrients, fixing soil nutrients, nitrogen metabolism, and other processes. Plant root systems are broadly classified into taproot-type system and beard-type root system. Taproot-type root system is found in dicotyledonous plants and consists of a taproot developed from a radicle and lateral roots that grow from the taproot at the lateral parts. In taproot-type root systems, the taproot extends vertically to the ground, providing support for the plant body and facilitating the absorption of water deep underground. In contrast, beard-type root system is found in monocots, wherein the seedroot corresponding to the taproots in taproot-type root systems develops very little, and primarily consists of nodal roots that grow from stem nodes. Beard-type root system is advantageous for growth in environments with abundant water and nutrients near the surface.

Since root system morphology can affect the efficiency of nutrient absorption from the root and the growth of aerial parts, it is important to develop techniques for controlling root system morphology.

Currently, root system morphology is altered mainly through breeding techniques using breed improvement, but it requires a huge amount of time and cost.

It is known that exogenous administration of auxin which is the plant hormone inhibits taproot elongation and promotes lateral root growth (non-patent literature 1). However, since the auxin's effect is potent, its use is limited. Furthermore, mepiquat chloride, which is a plant growth regulator used to increase cotton yields, is known to increase the number of lateral roots in cotton (non-patent literature 2). However, its rooting effect is weak in *Arabidopsis thaliana,* limiting its application to target plants.

On the other hand, 2-aminopimelic acid is a non-proteinogenic amino acid with two carboxyl groups and one amino group, and has been reported to be present in plants of the Pteridophyceae family (non-patent literature 3 and 4). However, its function has not yet been fully elucidated.

### Citation List

### Non-patent literature

Non-patent literature 1: Ping Song et al., 2021, Planta, 254:69.
Non-patent literature 2: Xiaojiao Chen et al., 2018, BMC Plant Biology, 18:361.
Non-patent literature 3: Noriaki Murakami and Shinichi Hatanaka, 1983, Phytochemistry, Vol. 22, No. 12, pp. 2735-2737.
Non-patent literature 4: Noriaki Murakami et al., 1985, Phytochemistry, Vol. 24, No. 24, pp. 2291-2294.

### Summary of Invention

### Technical Problem

The present invention aims to provide an accessible and effective means for controlling the root system morphology of plants.

### Solution to Problem

In order to solve the above problem, the present inventors focused on 2-aminopimelic acid. The present inventors found that cultivating plants in a medium containing 2-aminopimelic acid inhibited taproot growth, increased lateral root density, and promoted root hair growth. They also found that the rooting effect was promoted, leading to the completion of the present invention.

That is, the present invention includes the following.
[1] A root system morphology regulator of plants consisting of 2-aminopimelic acid or a salt or ester thereof.
[2] The root system morphology regulator according to [1], wherein the controlled root system morphology is at least one selected from fibrous root formation, promotion of root hair growth, and rooting promotion.
[3] The root system morphology regulator according to [1] or [2], wherein the plant is dicotyledonous plants.
[4] A composition comprising the root system morphology regulator according to any one of [1] to [3]
[5] A method for controlling root system morphology of plants, comprising a process of cultivating a plant with a medium comprising 2-aminopimelic acid or a salt or ester thereof.
[6] The method for controlling root system morphology of plants according to [5], wherein the controlled root system morphology is at least one selected from fibrous root formation, promotion of root hair growth, and rooting promotion.
[7] A method for producing a plant, comprising a process of cultivating a plant with a medium comprising 2-aminopimelic acid or a salt or ester thereof.
[8] The method according to any one of [5] to [7], wherein the medium is a liquid medium.

The present specification incorporates the contents disclosed in Japanese Patent Application No. 2023-122555, on which the priority of the present application is based.

### Effects of Invention

According to the present invention, the root of plants with taproot type root system morphology can be changed to a root similar to fibrous root type root system morphology.

### Brief Description of Drawings

[Figure 1] An image shows root system morphology during fledgling growth of *Arabidopsis thaliana* cultivated for 2 weeks in non-2-aminopimelic acid (2-APA)-containing MS medium (A) or 2-APA-containing MS medium (B).
[Figure 2] Graphs shows taproot length (A) and lateral root density (B) of *Arabidopsis thaliana* cultivated for 1 or 2 weeks in non-2-aminopimelic acid (2-APA)-containing MS medium or 2-APA-containing MS medium.
[Figure 3] Images show the root hair formation of *Arabidopsis thaliana* cultivated for two weeks in non-2-aminopimelic acid (2-APA)-containing MS medium (A) or 2-APA-containing MS medium (B).
[Figure 4] Images show root system morphology during fledgling growth of *Arabidopsis thaliana* ARF7 and ARF19 deletion mutants *(arf7-1 arf19-1*) cultivated in non-2-aminopimelic acid (2-APA)-containing MS medium (A) or 2-APA-containing MS medium (B).
[Figure 5] Graphs show taproot length (A) and lateral root density (B) of radish cultivated for 12 days in non-2-aminopimelic acid (2-APA) containing SkyGel (registered trademark) water-retaining medium or in 2-APA containing SkyGel water-retaining medium (n=4).
[Figure 6] Graphs show taproot length (A) and lateral root density (B) of broccoli plants cultivated for 12 days in non-2-aminopimelic acid (2-APA) containing SkyGel water-retaining medium or 2-APA containing SkyGel water-retaining medium (n = 7).
[Figure 7] Graphs show taproot length (A) and lateral root density (B) of komatsuna plants cultivated for 12 days in non-2-aminopimelic acid (2-APA) containing SkyGel water-retaining medium or 2-APA containing SkyGel water-retaining medium (n = 5).
[Figure 8] Graphs show taproot length (A) and lateral root density (B) of garden cress cultivated for 12 days in non-2-aminopimelic acid (2-APA) containing SkyGel water-retaining medium or 2-APA containing SkyGel water-retaining medium (n = 5).
[Figure 9] Graphs show taproot length (A) and lateral root density (B) of arugula cultivated for 12 days in non-2-aminopimelic acid (2-APA) containing SkyGel water-retaining medium or 2-APA containing SkyGel water-retaining medium (n = 5).
[Figure 10] Graphs show taproot length (A) and lateral root density (B) of mustard green cultivated for 12 days in non-2-aminopimelic acid (2-APA) containing SkyGel water-retaining medium or 2-APA containing SkyGel water-retaining medium (n = 5).
[Figure 11] Graphs show taproot length (A) and lateral root density (B) of sunflowers cultivated for 12 days in non-2-aminopimelic acid (2-APA) containing SkyGel water-retaining medium or 2-APA containing SkyGel water-retaining medium(n=4).
[Figure 12] Graphs show taproot length (A) and lateral root density (B) of cultivated white sesame for 12 days in non-2-aminopimelic acid (2-APA) containing SkyGel water-retaining medium or 2-APA containing SkyGel water-retaining medium (n = 2-4).
[Figure 13] Graphs show taproot length (A) and lateral root density (B) of cultivated red perilla for 12 days in non-2-aminopimelic acid (2-APA) containing SkyGel water-retaining medium or 2-APA containing SkyGel water-retaining medium (n = 5).
[Figure 14] Graphs show taproot length (A) and lateral root density (B) of cultivated green perilla for 11 days in non-2-aminopimelic acid (2-APA) containing SkyGel water-retaining medium or 2-APA containing SkyGel water-retaining medium (n = 5).
[Figure 15] Graphs show taproot length (A) and lateral root density (B) of red cabbage cultivated for 11 days in non-2-aminopimelic acid (2-APA) containing SkyGel water-retaining medium or 2-APA containing SkyGel water-retaining medium (n=5).
[Figure 16] Graphs show taproot length (A) and lateral root density (B) of alralfa cultivated for 12 daysin non-2-aminopimelic acid (2-APA) containing SkyGel water-retaining medium or 2-APA containing SkyGel water-retaining medium (n = 5).
[Figure 17] Graphs show taproot length (A) and lateral root density (B) of mungbean cultivated for 11 days in non-2-aminopimelic acid (2-APA) containing SkyGel water-retaining medium or 2-APA containing SkyGel water-retaining medium (n = 5).
[Figure 18] Graphs show taproot length (A) and lateral root density (B) of water morning glory plant cultivated for 12 days in non-2-aminopimelic acid (2-APA) containing SkyGel water-retaining medium or 2-APA containing SkyGel water-retaining medium (n=4-5).
[Figure 19] Graph show the relative lateral root density of *Arabidopsis thaliana* cultivated in MS medium containing different concentrations of 2-aminopimelic acid (2-APA) or non-2-aminopimelic acid containing MS medium. Data show average ± standard deviation (n = 7).

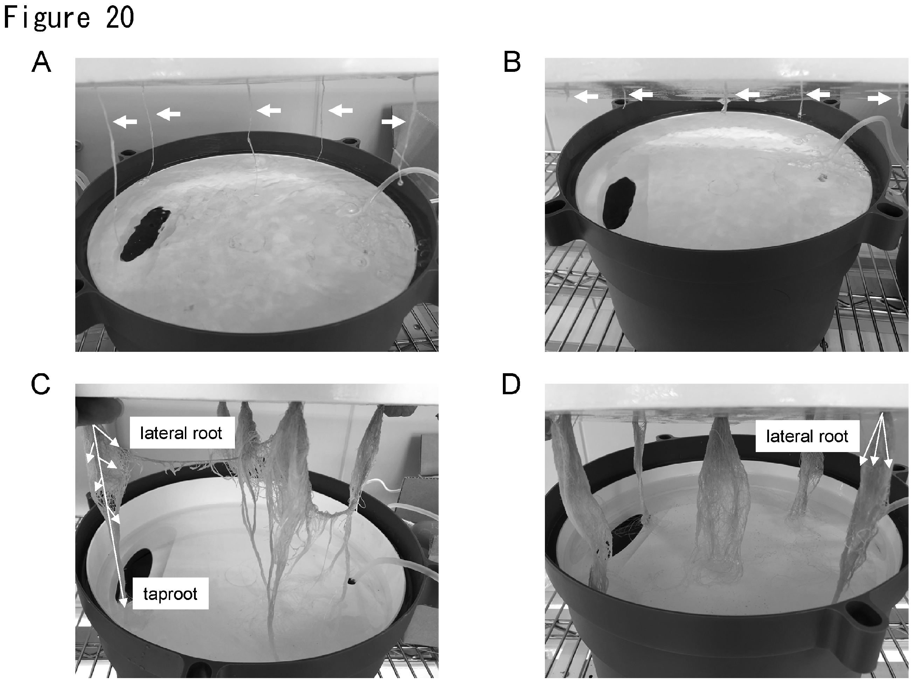

[Figure 20] Images show the root system morphology of komatsuna plants hydroponically cultured with a non-2-aminopimelic acid (2-APA) containing Hyponex solution or a 2-APA containing Hyponex solution. (A) root system morphology (arrow) of komatsuna plant hydroponically cultured with a non-2-aminopimelic acid (2-APA) containing Hyponex solution 12 days after sowing. (B) Root system morphology (arrow) of komatsuna plant hydroponically cultured with a 2-aminopimelic acid (2-APA) containing Hyponex solution 12 days after sowing. (C) Root system morphology (arrow) of komatsuna plant hydroponically cultured with a non-2-aminopimelic acid (2-APA) containing Hyponex solution 24 days after sowing. (D) Root system morphology (arrow) of komatsuna plant hydroponically cultured with a 2-aminopimelic acid (2-APA) containing Hyponex solution 24 days after sowing.
[Figure 21] Figure shows (A) the fresh weight of the aerial part and (B) the fresh weight of the aerial part/root organ of komatsuna plant that were hydroponically cultured for approximately one month in a non-2-aminopimelic acid (2-APA) containing Hyponex solution or a 2-aminopimelic acid (2-APA) Hyponex solution 40 days after sowing, (n=5).
[Figure 22] Images show root system morphology during fledgling growth of *Arabidopsis thaliana* cultivated for 2 weeks in non-2-aminopimelic acid (2-APA)-containing MS medium (A), DL-2-APA-containing MS medium (B), or L-2-APA-containing MS medium (C).
[Figure 23] Graphs showing taproot length (A) and lateral root density (B) of *Arabidopsis thaliana* cultivated for 2 weeks in non-2-aminopimelic acid (2-APA)-containing MS medium, DL-2-APA-containing MS medium, or L-2-APA-containing MS medium.

### Description of Embodiments

The present invention is described in detail as below.

The present invention relates to the use of 2-aminopimelic acid or a salt or ester thereof, for controlling root system morphology of plants. The present invention particularly relates to the use of 2-aminopimelic acid or a salt or ester thereof, for fibrous root formation, promotion of root hair growth, and rooting promotion of plants.

### (1) Root System Morphology Regulator

The present invention provides a root system morphology regulator of plants.

In this specification, "root system morphology control" means changing root system morphology to a desired form. The root system morphology control is particularly at least one selected from fibrous root formation, promotion of root hair growth, and rooting promotion. The root system morphology control may be, for example, fibrous root formation and promotion of root hair growth, fibrous root formation and rooting promotion, or fibrous root formation, promotion of root hair growth, and rooting promotion.

In this specification, "fibrous root formation" means that the root system morphology of plants change to a beard-type root system morphology or similar form thereof, particularly that taproot growth is inhibited and lateral root density is increased. In this specification, "inhibiting taproot growth" includes shortening the taproot length and inhibiting taproot elongation. In this specification, "lateral root density" means the number of lateral roots (particularly the number of primary lateral roots) per unit length arising from the taproot, and can be calculated by dividing the taproot length by the number of lateral roots.

In this specification, " promotion of root hair growth" means increasing the number and/or length of root hairs. In this specification, "root hair" means a hair-like organ that grows from part of the root epidermis. The root hair increases the surface area of the root, improving the absorption efficiency of water and nutrients, and also plays a role in fixing the root to the soil.

In this specification, "rooting promotion" means promoting root formation, particularly increasing the number of lateral roots.

In this specification, "root system morphology regulator" refers to an agent consisting of 2-aminopimelic acid a salt or ester thereof, preferably 2-aminopimelic acid or the salt thereof, which has the root system morphology control effect of plants.

As shown in the examples below, 2-aminopimelic acid inhibits taproot growth and increases lateral root density (*i.e.,* promotes fibrous root formation of plants). 2-aminopimelic acid also promotes root hair growth and increases the number of lateral roots. Therefore, 2-aminopimelic acid and its analogs, such as salts and esters of 2-aminopimelic acid, can be used as active ingredients in the root system morphology regulator of the present invention.

2-aminopimelic acid is a compound represented by formula (I) below.

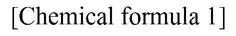

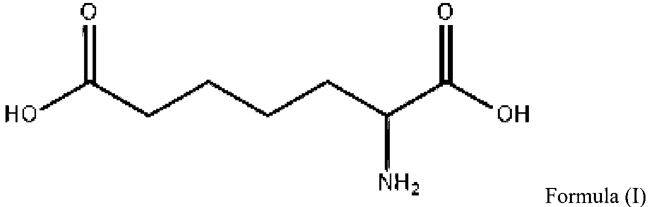

2-aminopimelic acid may be a D-isomer, an L-isomer, or an equal mixture thereof (DL-isomer), but is preferably an L-isomer or a DL-isomer, more preferably an L-isomer.

The salt of 2-aminopimelic acid may be any agronomically acceptable salt, and is not particularly limited to, for example, acid addition salts, metal salts, ammonium salts, amine salts, and others. Acid addition salts include, for example, hydrochloride, phosphate, nitrate, sulfate, acetate, propionate, butyrate, valerate, citrate, fumarate, maleate, malate, and others. Metal salts include magnesium salts, calcium salts, and other alkaline earth metal salts, aluminum salts, and zinc salts.

The ester of 2-aminopimelic acid may be any agronomically acceptable ester, and is not particularly limited, but may be, for example, an ester in which one or both hydrogen atoms of the two carboxyl groups comprised in 2-aminopimelic acid are substituted with an alkyl group. Examples of the alkyl group include a C1-C5 alkyl group (a linear or branched alkyl group having 1 to 5 carbon atoms), for example, a methyl group (-CH₃), an ethyl group (-C₂H₅), a propyl group (-C₃H₇), a butyl group (-C₄H₉), and a pentyl group (-C₅H₁₁).

A commercially available 2-aminopimelic acid may be used, and can be obtained, for example, from Fujifilm Wako Pure Chemical Industries, Ltd., Tokyo Chemical Industry Co., Ltd., and others. A salt or ester of 2-aminopimelic acid can be obtained from 2-aminopimelic acid by a conventional method. An isolated or purified 2-aminopimelic acid or a salt or ester thereof, may be used.

The target plants of the root system morphology regulator of the present invention may be any plants including angiosperms (dicotyledonous plants, monocots), gymnosperms, ferns, bryophytes, and algae, but are preferably plants with taproot-type root systems, for example, dicotyledonous plants. The plants may be ornamental flowering plants, edible vegetables, fruits, and other agricultural crops. Dicotyledonous plants include, for example, Brassicaceae plants (for example, *Arabidopsis thaliana,* radish, broccoli, komatsuna, garden cress, arugula, mustard, cabbage, Chinese cabbage, mizuna, bok choy, and others), Asteraceae plants (for example, chrysanthemum, sunflower, lettuce, and others), Pesaceae plants (for example, sesame, and others), Lamiaceae plants (for example, perilla, sage, thyme, basil, mint, lavender, lemon balm, rosemary, and others), Legume plants (for example, alfalfa, mungbean, adzuki bean, green beans, broad beans, pea, soybean, chickpea, peanut, and others), Convolvulaceae plants (for example, water morning glory, sweet potato, and others), Cucurbitaceae plants (for example, cucumber, pumpkin, watermelon, melon, zucchini, and others), Rosaceae plants (for example, strawberry, peach, pear, apple, almond, and others), Apiaceae plants (for example, carrot, celery, parsley, and others), and Solanaceae plants (for example, eggplant, tomato, potato, bell pepper, and others). It is preferably, but not limited to, *Arabidopsis thaliana,* radish (*Raphanus sativus var. Longipinnatus*), broccoli (*Brassica oleracea var. italica*), komatsuna (*Brassica rapa var. perviridis*), garden cress (*Lepidium sativum*), arugula (*Eruca vesicaria*), mustard (*Brassica juncea*), sunflower (*Helianthus annuus*), white sesame (*Sesamum indicum*), red perilla (*Perilla frutescens var. crispa f. purpurea),* green perilla (P*erilla frutescens var.crispa f. viridis),* red cabbage (*Brassica oleracea var. capitata),* alfalfa (*Medicago sativa),* mungbean (*Vigna radiata),* or water morning glory (*Ipomoea aquatica).*

Whether 2-aminopimelic acid a salt or ester thereof has the effect of promoting fibrous root formation of plants can be evaluated by comparing the taproot length and lateral root density of plants cultivated in a medium containing 2-aminopimelic acid a salt or ester thereof with that cultivated in a medium not containing 2-aminopimelic acid a salt or ester thereof. For example, plant seeds are sowed in a medium containing 2-aminopimelic acid a salt or ester thereof, or in a medium not containing 2-aminopimelic acid a salt or ester thereof, and cultivated for a specified period (for example, 2 weeks), and the taproot length and number of lateral roots (particularly the number of primary lateral roots) of the resulting plant body are measured. When the average taproot length of plants cultivated in a medium containing 2-aminopimelic acid a salt or ester thereof is reduced by 10% or more and the average lateral root density is increased by 10% or more compared to plants cultivated in a medium not containing 2-aminopimelic acid a salt or ester thereof, it can be determined that 2-aminopimelic acid, a salt, or ester thereof has the effect of promoting fibrous root formation of plants.

Whether 2-aminopimelic acid a salt or ester thereof has the effect of promoting root hair growth can be evaluated by comparing the number and/or length of root hairs of plants cultivated in a 2-aminopimelic acid or a salt or ester thereof containing medium , with that of plants cultivated in a non-2-aminopimelic acid or a salt or ester thereof-containing medium. For example, plant seeds are sowed in the 2-aminopimelic acid or a salt or ester thereof-containing medium, or in the non-2-aminopimelic acid or a salt or ester thereof-containing medium, and cultivated for a predetermined period (for example, 2 weeks). When the average number and/or length of root hairs of plants cultivated in the 2-aminopimelic acid or a salt or ester thereof-containing medium increases by 10% or more compared to plants cultivated in the non-2-aminopimelic acid or a salt or ester thereof-containing medium, it can be determined that 2-aminopimelic acid a salt or ester thereof has the effect of promoting root hair growth.

Whether 2-aminopimelic acid a salt or ester thereof has a root promoting effect can be evaluated by comparing the number of lateral roots (particularly the number of primary lateral roots) of plants cultivated in a 2-aminopimelic acid or a salt or ester thereof-containing medium with that cultivated in a non-2-aminopimelic acid or a salt or ester thereof-containing. For example, plant seeds are sowed in the 2-aminopimelic acid or a salt or ester thereof-containing medium, or in the non-2-aminopimelic acid or a salt or ester thereof-containing medium, and cultivated for a predetermined period (for example, 2 weeks), and the number of lateral roots of the resulting plant body is measured. When the average number of lateral roots of plants cultivated in the 2-aminopimelic acid or a salt or ester thereof-containing medium is increased by 10% or more compared to plants cultivated in the non-2-aminopimelic acid or a salt or ester thereof-containing medium, it can be determined that 2-aminopimelic acid or a salt or an ester thereof has a root promoting effect.

The root system morphology regulator of the present invention can trivialize the rhizosphere and reduce root gross weight by suppressing taproot growth, while altering root system morphology to increase nutrient absorption efficiency from the root. As a result, the root system morphology regulator of the present invention can trivialize the rhizosphere and reduce root gross weight while maintaining aerial growth. This leads to savings in the amount of soil or water used for plant cultivation, and ultimately savings in cultivation space. Therefore, the root system morphology regulator of the present invention is particularly useful in nutriculture such as hydroponics cultivation and others, where savings in soil or water used for cultivation and cultivation space are important.

The root system morphology regulator of the present invention can also be used, for example, for rooting promotion of cuttings or cutting seedlings. Since the root system morphology regulator of the present invention has a growth promoting effect of root hairs and a root promoting effect, it can also be used, for example, to promote the establishment of plants after transplanting.

Currently, commercially available rooting agents that promote root development include "hormonal rooting agents" comprising auxins as active ingredients, and "activator-type rooting agents" comprising vitamins, minerals, and other active ingredients to improve nutritional status (https://www.sc-engei.co.jp/guide/detail/1421, https://www.sun-g-orient.co.jp/hakkonryoku/). However, hormonal rooting agents have issues such as environmental concerns due to the use of high concentrations of plant hormones, and their extremely strong effects limit their use to soaking for several days. Furthermore, activator-type rooting agents have issues with optimizing their functional efficiency as rooting agents because the nutritional requirements vary depending on the cultivation environment and plant variety.

Because the root system morphology regulator of the present invention is not a plant hormone, there are no limitations on its usage, unlike hormonal rooting agents comprising auxins as active ingredients. The root system morphology regulator of the present invention can be used more easily than hormonal rooting agents, by only adding it to a plant cultivation medium. Furthermore, as shown in the examples below, the mechanism of the root system morphology regulator of the present invention is thought to be similar to that of hormonal rooting agents, and therefore the root system morphology regulator of the present invention can promote plant root development more directly than activator-type rooting agents comprising vitamins, minerals, and others as active ingredients.

In one embodiment, the root system morphology regulator of the present invention is a fibrous root formation agent for dicotyledonous plants, for example, Brassicaceae plants, Asteraceae plants, Pesaceae plants, Lamiaceae plants, Legume plants, or Convolvulaceae plants.

In one embodiment, the root system morphology regulator of the present invention can be an agent for promoting root hair growth in Brassicaceae plants.

In one embodiment, the root system morphology regulator of the present invention can be a rooting promoter for Asteraceae or Brassicaceae plants, for example, sunflower or cabbage.

### (2) Composition.

The present invention provides a composition (hereinafter, also called "composition of the present invention") comprising 2-aminopimelic acid or a salt or ester thereof, or the root system morphology regulator of the present invention. The composition of the present invention can be used for at least one selected from control of plant root system morphology, particularly plant fibrous root formation, root hair growth promotion, and rooting promotion.

The composition of the present invention comprises 2-aminopimelic acid or a salt or ester thereof, preferably 2-aminopimelic acid or a salt thereof, as an essential active ingredient.

The composition of the present invention may further comprise other plant growth regulators or essential elements such as hydrogen (H), oxygen (O), carbon (C), nitrogen (N), phosphorus (P), potassium (K), magnesium (Mg), calcium (Ca), sulfur (S), iron (Fe), manganese (Mg), zinc (Zn), boron (B), molybdenum (Mo), copper (Cu), chlorine (Cl), and nickel (Ni) and others.

In addition to the above components, the composition of the present invention may further comprise agriculturally acceptable additives, for example, carriers (such as solid or liquid carriers), excipients, diluents, disintegrants, binders, coating agents, lubricants, glidants, emulsifiers, surfactants, solubilizers, suspending agents, preservatives, buffers, or pH adjusters, and others.

The composition of the present invention may be formulated into any dosage form, for example, solid dosage forms such as dusts, granules, powders, wettable powders, water-soluble and others, liquid preparations such as emulsions, solutions, oils and others, aerosols, pastes, sprays and others. The composition of the present invention may, for example, be a dissolution of 2-aminopimelic acid or a salt or ester thereof, in a suitable solution. Suitable solutions include water (including sterile water, deionized water, and ultrapure water), buffer solutions, liquid media, and others.

The composition of the present invention can be used, for example, by adding it to a medium for growing plants. The content of 2-aminopimelic acid a salt or ester thereof in the composition of the present invention is not limited, as long as a final concentration of 2-aminopimelic acid or a salt or ester thereof can provide effective concentration for controlling root system morphology (fibrous root formation, promotion of root hair growth, rooting promotion, etc.) of plants when the composition of the present invention is added to a medium and others. The concentration of 2-aminopimelic acid or a salt or ester thereof in the composition of the present invention is not limited, and may be, for example, 0.1 µM or more, 1 µM or more, 10 µM or more, 50 µM or more, 100 µM or more, 1 mM or more, 10 mM or more, 1 mM or less, 500 mM or less, 100 mM or less, for example, 10 µM to 100 mM, 50 µM to 100 mM, 100 µM to 100 mM, 1 mM to 100 mM, 10 mM to 100 mM, 10 µM to 50 mM, 50 µM to 50 mM, 100 µM to 50 mM, 1 mM to 50 mM, or 10 mM to 50 mM. The composition of the present invention can be added to a medium so that the concentration of 2-aminopimelic acid or a salt or ester thereof in the medium is, for example, 0.1 µM or more, 1 µM or more, 10 µM or more, 50 µM or more, 60 µM or more, 70 µM or more, 80 µM or more, or 90 µM or more, 10 mM or less, 1 mM or less, 500 µM or less, 400 µM or less, 300 µM or less, 200 µM or less, 150 µM or less, 140 µM or less, 130 µM or less, 120 µM or less, or 110 µM or less, for example, 0.1 µM to 10 mM, 1 µM to 1 mM, 50 µM to 500 µM, 50 µM to 150 µM, or 90 µM to 110 µM.

Targets for administration of the composition of the present invention are the same as those for administration of the root system morphology regulator of the present invention described above, for example, angiosperm (dicotyledonous plants, monocots), gymnosperms, ferns, bryophytes, and algae. Dicotyledonous plants are preferred, and plants of Brassicaceae plants, Asteraceae plants, Pesaceae plants, Lamiaceae plants, Legume plants, Convolvulaceae plants, Cucurbitaceae plants, Rosaceae plants, Apiaceae plants, or Solanaceae plants are more preferred. *Arabidopsis thaliana,* radish, broccoli, komatsuna, garden cress, arugula, mustard, sunflower, white sesame, red perilla, green perilla, red cabbage, alfalfa, mungbean, or water morning glory are even more preferred.

### (3) Method for controlling root system morphology.

The present invention relates to a method for controlling root system morphology of plants (sometimes called "control method of root system morphology of the present invention"), which comprises a process of cultivating plants with a medium comprising 2-aminopimelic acid or a salt or ester thereof, or the root system morphology regulator of the present invention. In the control method of root system morphology of the present invention, the control of root system morphology can be, for example, at least one selected from fibrous root formation, promotion of root hair growth, and rooting promotion.

In this specification, "medium" refers to a medium used for plant cultivation and includes liquid medium and solid medium. The liquid medium may be any suitable medium for plant cultivation, and is not particularly limited, but preferably contains the essential elements hydrogen (H), oxygen (O), carbon (C), nitrogen (N), phosphorus (P), potassium (K), magnesium (Mg), calcium (Ca), sulfur (S), iron (Fe), manganese (Mg), zinc (Zn), boron (B), molybdenum (Mo), copper (Cu), chlorine (Cl), nickel (Ni), and others. The liquid medium may contain, for example, inorganic salts (for example, potassium nitrate, ammonium nitrate, magnesium sulfate, iron sulfate, manganese sulfate, zinc sulfate, copper sulfate, potassium phosphate, sodium molybdate, ethylenediaminetetraacetic acid disodium salt, boric acid, calcium chloride, cobalt chloride, potassium iodide, and others), sugars, amino acids, vitamins, and others. Examples of suitable liquid medium include Murashige-Skoog (MS) medium, White's medium, commercially available Hyponex (registered trademark) solution, and others. Examples of solid medium include culture soil, liquid medium solidified by adding gelling agents such as agar, gelatin, gellan gum, cross-linked polyacrylic acid polymer and others, and gravel, sand, vermiculite, perlite, rock wool, rice husks, bark, peat moss, coconut shell, polyester, urethane and others. Liquid medium may also be added to the solid medium.

2-aminopimelic acid or a salt or ester thereof may be added to the medium in an amount effective for controlling the root system morphology of the plant (fibrous root formation, promotion of root hair growth, rooting promotion, and others). For example, 2-aminopimelic acid or a salt or ester thereof can be added to the medium so that the concentration in the medium is 0.1 µM or more, 1 µM or more, 10 µM or more, 50 µM or more, 60 µM or more, 70 µM or more, 80 µM or more, 90 µM or more, 10 mM or less, 1 mM or less, 500 µM or less, 400 µM or less, 300 µM or less, 200 µM or less, 150 µM or less, 140 µM or less, 130 µM or less, 120 µM or less, or 110 µM or less, for example, 0.1 µM to 10 mM, 1 µM to 1 mM, 50 µM to 500 µM, 50 µM to 150 µM, or 90 µM to 110 µM.

Plants to be cultivated using the control method of root system morphology of the present invention are similar to the targets of administration of the root system morphology regulator of the present invention described above. Examples include angiosperm (dicotyledonous plants, monocots), gymnosperms, ferns, bryophytes, and algae. Dicotyledonous plants are preferred, and more preferred are plants of the Brassicaceae plants, Asteraceae plants, Pesaceae plants, Lamiaceae plants, Legume plants, Convolvulaceae plants, Cucurbitaceae plants, Rosaceae plants, Apiaceae plants, or Solanaceae plants. Even more preferred are *Arabidopsis thaliana,* radish, broccoli, komatsuna, garden cress, arugula, mustard, sunflower, white sesame, red perilla, green perilla, red cabbage, alfalfa, mungbean, and water morning glory. In this specification, unless the context requires otherwise, "plant" includes the whole or part of the plant body, seed and others. The part of the plant body, for example, includes leaves, stem, root, stem tip, axillary bud, ovary, embryo, pollen, anther, tissue fragments comprising these, cultured cells (for example, callus), and others.

In the control method of root system morphology of the present invention, cultivation with a medium comprising 2-aminopimelic acid or a salt or ester thereof may be performed at any suitable time, for example, during seedling raising, after planting, or between sowing and harvesting the plant.

In the control method of root system morphology of the present invention, the form of cultivation is not particularly limited, for example, it may be soil cultivation, nutrient solution soil cultivation, or nutriculture (including hydroponics, rockwool cultivation, coco peat cultivation, and others).

Cultivation conditions may be determined appropriately depending on the type of plants. The cultivation temperature may be, for example, 15°C to 30°C, 20°C to 30°C, or 20°C to 25°C. The light/dark cycle may be, for example, a 6-18 hour, 10-18 hour, 14-18 hour, or 16 hour light period and a 6-18 hour, 6-14 hour, 6-10 hour, or 8 hour dark period. The cultivation period may be, for example, 1 day or more, 3 days or more, 1 week or more, 2 weeks or more, 1 year or less, 6 months or less, 3 months or less, 1 month or less, 4 weeks or less, or 3 weeks or less, for example, 1 week to 1 year, 1 week to 6 months, 1 week to 3 months, 1 week to 1 month, 1 week to 3 weeks, 2 weeks to 1 year, 2 weeks to 6 months, 2 weeks to 3 months, 2 weeks to 1 month, or 2 weeks to 3 weeks. When cultivating komatsuna hydroponically, for example, the cultivation temperature may be 20°C to 25°C, the photoperiodic conditions may be a light period of approximately 16 hours and a dark period of approximately 8 hours, and the cultivation period may be 2 weeks to 1 month.

In one embodiment, the cultivation process may comprise a process of sowing plant seeds in a medium containing 2-aminopimelic acid or a salt or ester thereof, cultivating the seedling raising, and a process of planting the resulting seedlings in another medium containing 2-aminopimelic acid or a salt or ester thereof, and cultivating the plant.

### (4) Method for producing plants.

The present invention relates to a plant cultivation method (sometimes called " production method of the present invention") comprising a process of cultivating a plant with a medium comprising 2-aminopimelic acid or a salt or ester thereof.

In the production method of the present invention, the process of cultivating with a medium comprising 2-aminopimelic acid or a salt or ester thereof is the same as the control method of root system morphology of the present invention.

In the production method of the present invention, cultivating a plant in a medium comprising 2-aminopimelic acid or a salt or ester thereof can control the root system morphology of the plants, particularly, can bring about at least one selected from fibrous root formation, promotion of root hair growth, and rooting promotion.

The production method of the present invention may or may not further comprise a process of cultivating a plant in a non-2-aminopimelic acid or a salt or ester thereof-containing medium.

According to the production method of the present invention, the effect of 2-aminopimelic acid a salt, or ester thereof can change the root system morphology of plants to fibrous root formation. As a result, the efficiency of plant cultivation can be improved in environments where a hair root-type root system is more favorable for growth than a taproot-type root system (for example, environments with abundant moisture and nutrients near the surface). For example, in hydroponics, the amount of soil or water used for cultivation and the cultivation space can be saved.

### Examples.

The present invention will be explained in more detail using examples. However, the technical scope of the present invention is not limited to these examples.

### Example 1: Effect of 2-aminopimelic acid on root system morphology of Arabidopsis thaliana

To investigate the effect of 2-aminopimelic acid on the root system morphology of *Arabidopsis thaliana,* following experiments were performed.

Murashige and Skoog Plant Salt Mixture (Fujifilm Wako Pure Chemical Corporation) was dissolved in distilled water, to which 0.1% MES (Nacalai Tesque, Inc.) and 0.2-0.4% Gellan Gum (Fujifilm Wako Pure Chemical Corporation) were added, followed by dry heat sterilization to prepare MS (Murashige and Skoog) medium. DL-2-aminopimelic acid (CAS RN: 627-76-9, Tokyo Chemical Industry Co., Ltd.) was added in an appropriate amount to the MS medium after dry heat sterilization to prepare 2-aminopimelic acid-containing MS medium at a final concentration of 100 µM.

Seeds of Columbia-0 (Col-0), which was the wild-type strain of *Arabidopsis thaliana,* were surface-sterilized and sowed in 100 µM 2-aminopimelic acid-containing MS medium in a square petri dish (Eiken Chemical Co., Ltd.). As a control, sterilized seeds were sowed in non-2-aminopimelic acid-containing MS medium.

The plants were left at 4°C under dark conditions for three days, starting from the day of sowing, for low temperature treatment. The plants were then cultivated under photoperiodic conditions of 22°C, 16 hours of light, and 8 hours of dark. After one and two weeks, the taproot length of the plants was measured using Vernier calipers, and the number of lateral roots (number of primary lateral roots) of the plants was counted. The number of lateral roots divided by the taproot length was used to calculate the lateral root density.

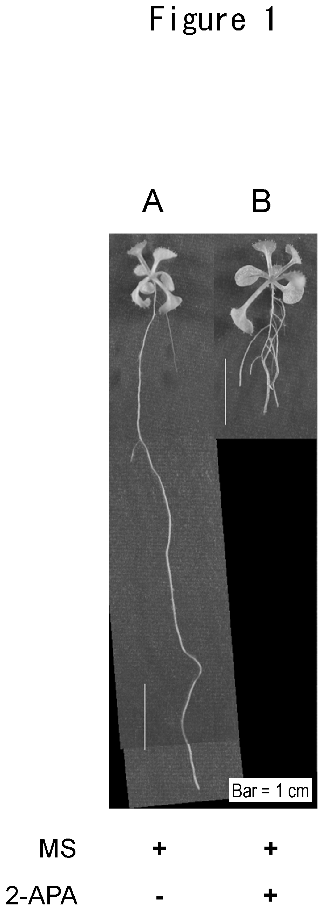

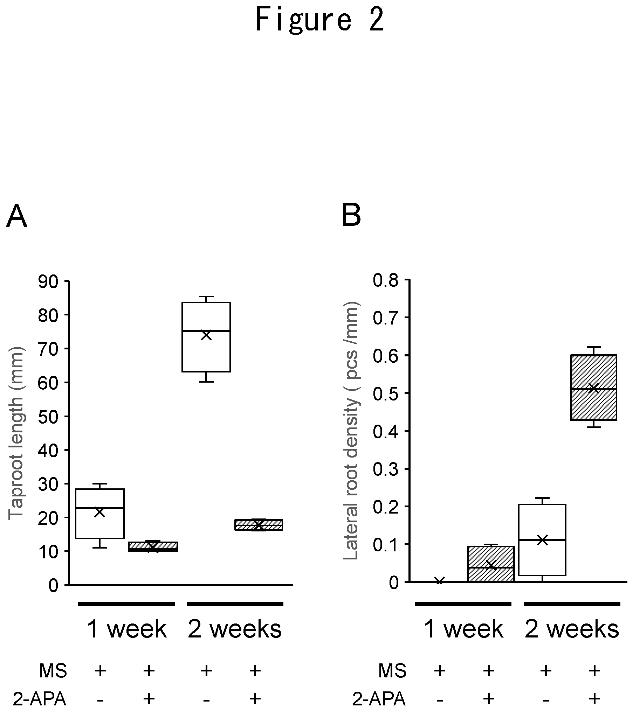

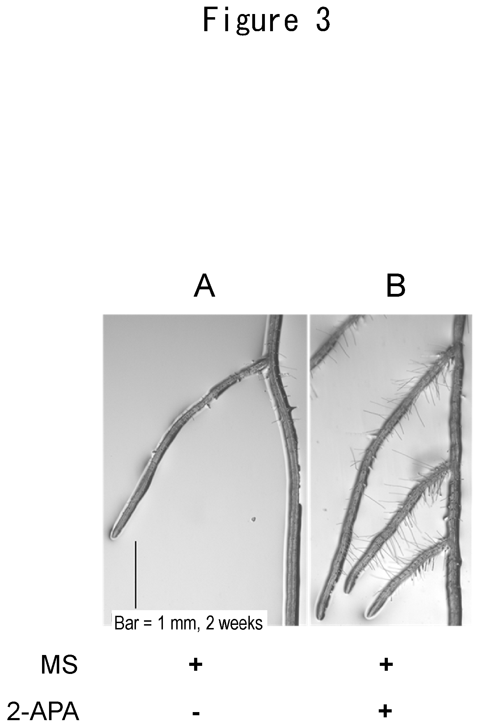

The results are shown in Figures 1 to 3. Compared with non-2-aminopimelic acid-containing MS medium, 2-aminopimelic acid-containing MS medium reduced taproot length while increasing lateral root density (Figures 1 and 2). These results suggest that cultivation in the presence of 2-aminopimelic acid transforms taproot-type root systems into roots with a similar form of hair-type root systems, i.e., fibrous root formation. Furthermore, 2-aminopimelic acid-containing MS medium increased the number and length of root hairs compared with 2-aminopimelic acid-containing MS medium (Figure 3). These results suggest that 2-aminopimelic acid promotes root hair growth.

### Example 2: Effect of 2-aminopimelic acid on root system morphology of ARF7 and ARF19 deletion mutants of Arabidopsis thaliana.

The changes in root system morphology of *Arabidopsis thaliana* observed in the presence of 2-aminopimelic acid in Example 1 were similar to those observed in the presence of the plant hormone auxin (Ping Song et al., 2021, Planta, 254:69, pp. 1-13).

Therefore, to examine the relationship between 2-aminopimelic acid and auxin biosynthesis, we performed following experiments using *Arabidopsis thaliana* lacking the auxin-response genes ARF7 and ARF19 (*arf7-1 arf19-1;* Arabidopsis Biological Resource Center).

MS medium containing or not containing 100 µM 2-aminopimelic acid, subjected to low temperature treatment, and cultivated for 2 weeks, after which root system morphology was observed. The medium, sowing, low temperature treatment, and cultivation method used were as described in Example 1.

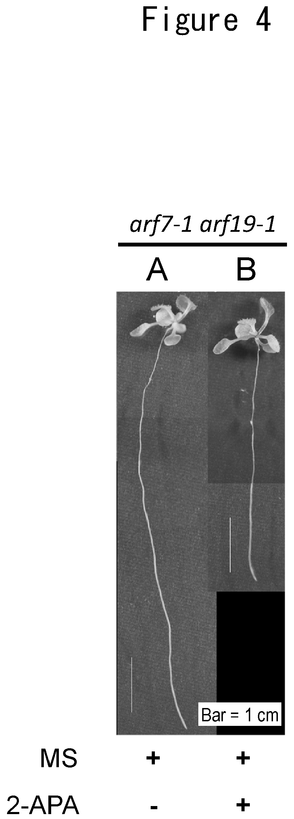

The results are shown in Figure 4. In the *arf7-1 arf19-1* strain, 2-aminopimelic acid had no effect on root system morphology. This result suggests that 2-aminopimelic acid alters root system morphology by regulating the biosynthesis or action of auxin.

### Example 3: Effect of 2-aminopimelic acid on the root system morphology of various plants.

To investigate the effect of 2-aminopimelic acid on the root system morphology of various plants other than *Arabidopsis thaliana,* following experiments were performed.

An appropriate amount of SkyGel (registered trademark) (Maybiol Co., Ltd.) and 100 ml of RO water were added to a plant box (AS ONE) to prepare SkyGel water-retaining medium. Furthermore, an appropriate amount of SkyGel and 100 ml of 100 µM DL-2-aminopimelic acid (CAS RN: 627-76-9, Tokyo Chemical Industry Co., Ltd.) solution were added to the plant box to prepare 100 µM 2-aminopimelic acid-containing SkyGel water-retaining medium.

Each seed (Nakahara Seed Co., Ltd.) of Radish (Raphanus sativus var. Longipinnatus), broccoli (Brassica oleracea var. italica), komatsuna (Brassica rapa var. perviridis), garden cress (Lepidium sativum), arugula (Eruca vesicaria), mustard (Brassica juncea) and red cabbage (Brassica oleracea var. capitata) from Brassicaceae plants, sunflower (Helianthus annuus) from Asteraceae plants, white sesame (Sesamum indicum) from Pedaliaceae plants, red perilla (Perilla frutescens var. crispa f.purpurea) and green perilla (Perilla frutescens var. crispa) from Lamiaceae plants, alfalfa (Medicago sativa) and mungbean (Vigna radiata) from Fabaceae, and water morning glory (Ipomoea aquatica) from Convolvulaceae plants were disinfected for several seconds in 80% ethanol and then sowed in 100 µM 2-aminopimelic acid-containing SkyGel water-retaining medium. Control seeds were also sowed in non-2-aminopimelic acid-containing SkyGel water-retaining medium. The plants were then cultivated under photoperiodic conditions at 23°C with a 16-hour light period and an 8-hour dark period. After two weeks, the taproot length of each plant was measured using Vernier calipers, and the number of lateral roots (number of primary lateral roots) was also counted. Lateral root density was calculated by dividing the number of lateral roots by the taproot length.

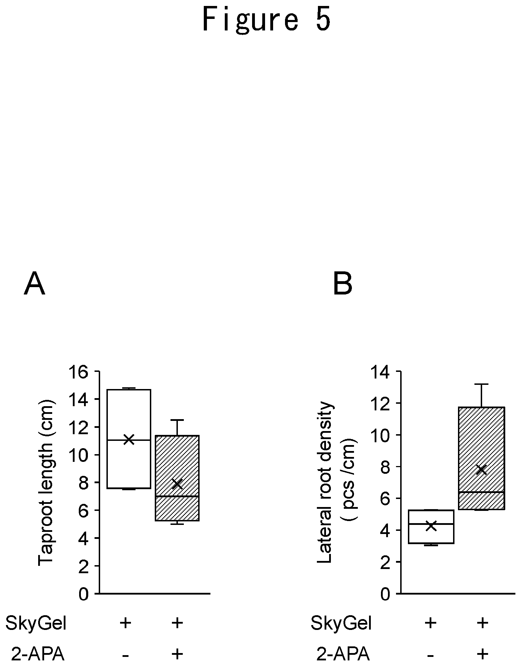

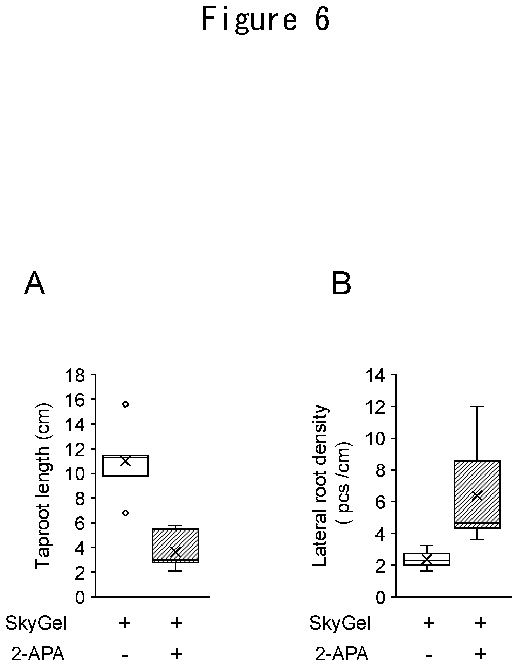

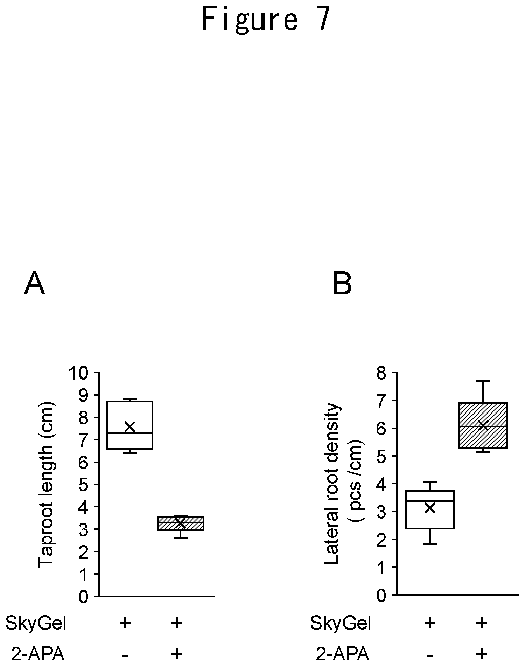

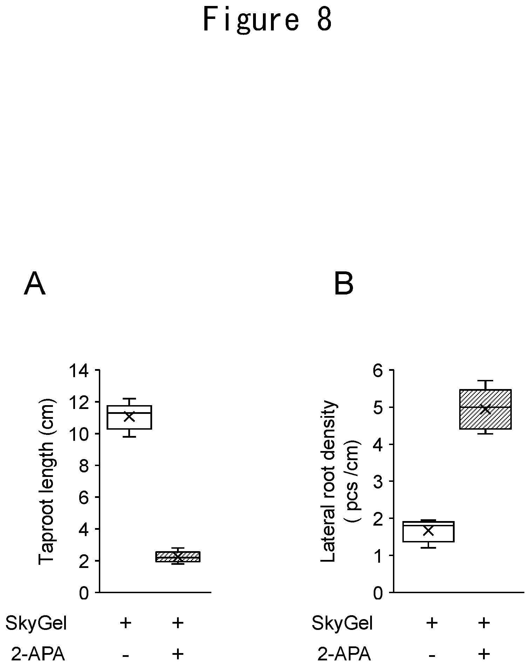

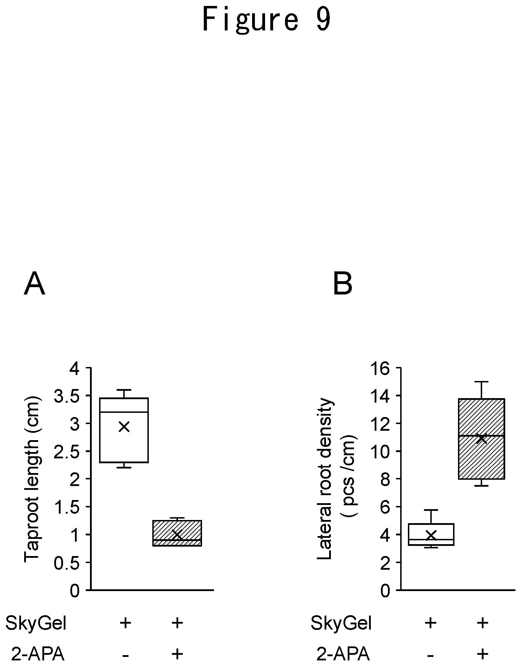

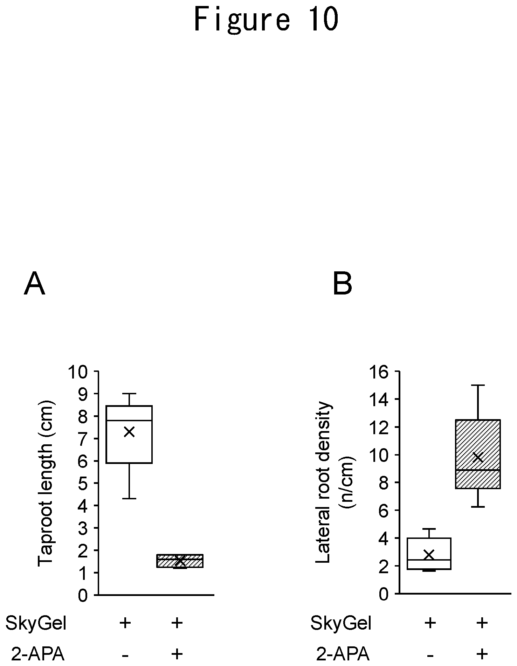

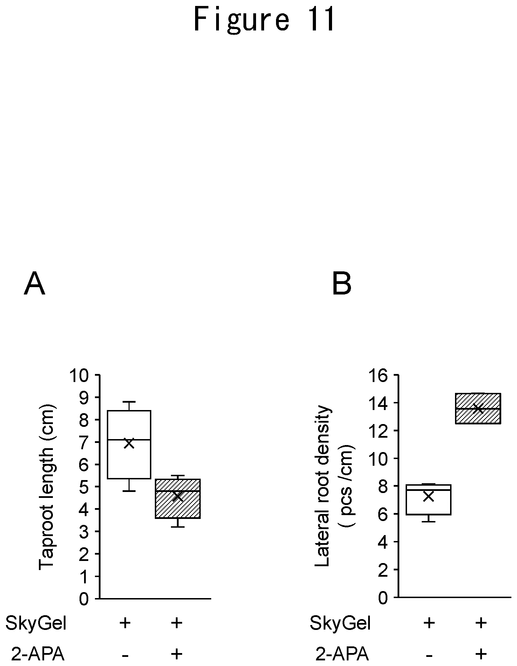

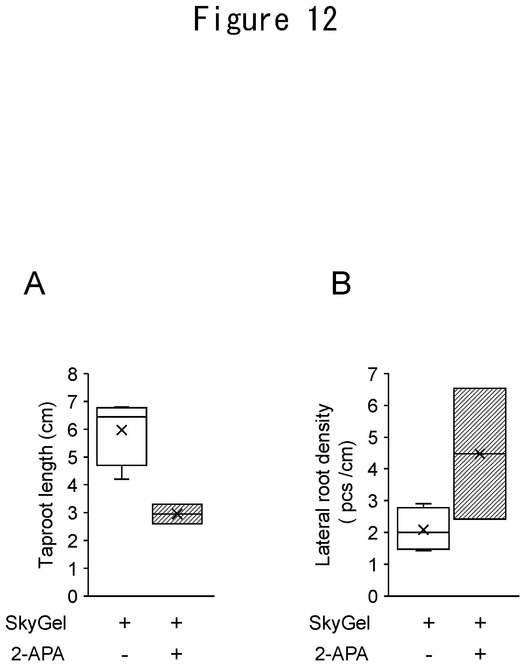

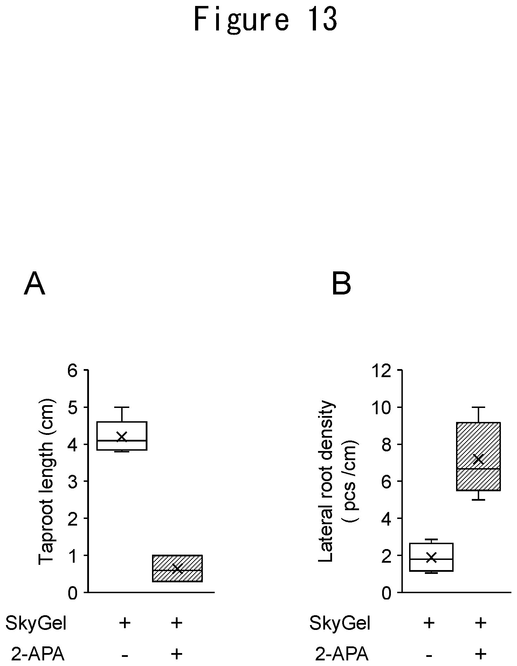

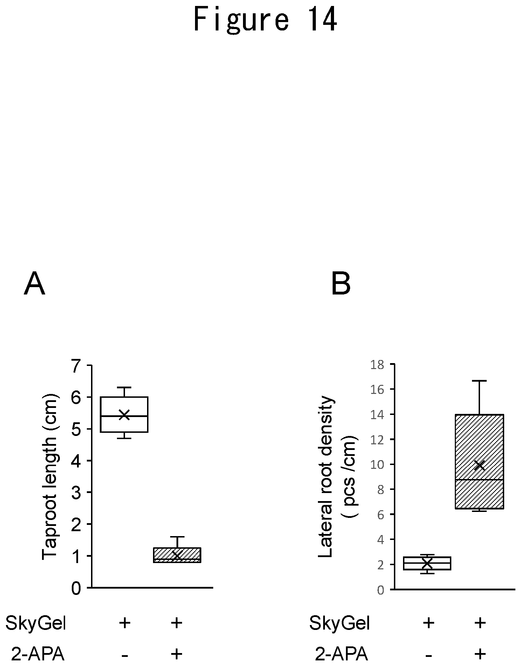

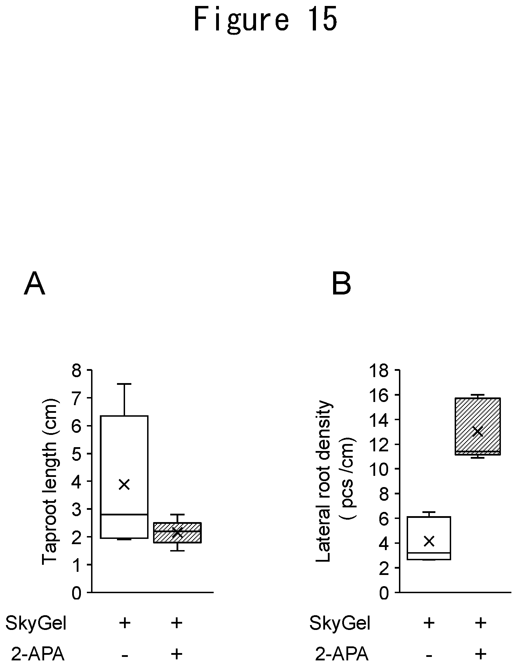

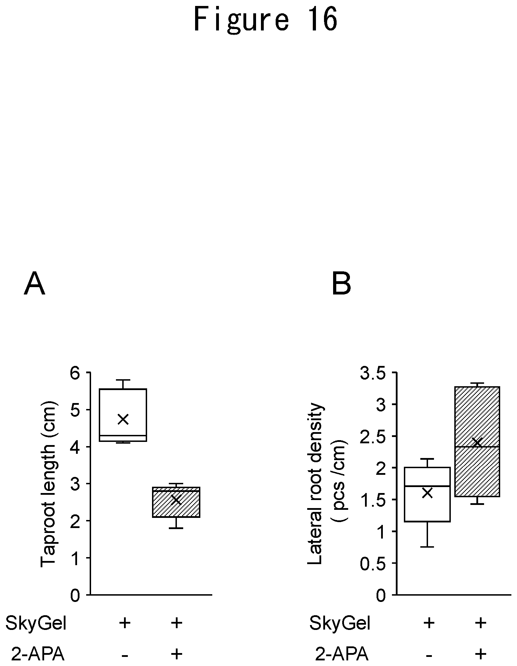

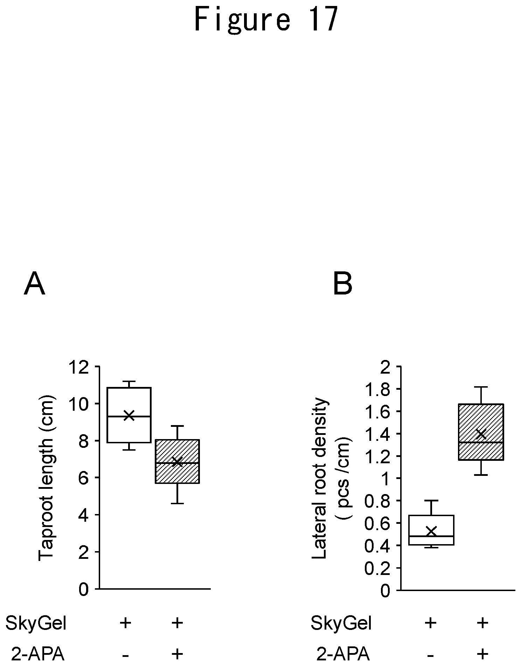

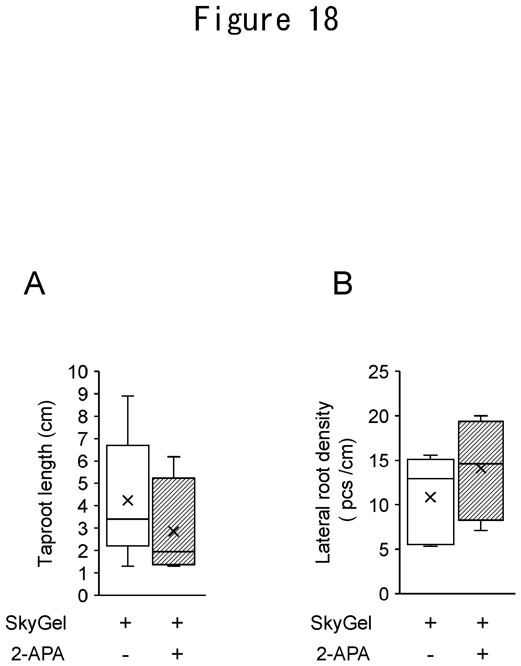

The results are shown in Figures 5 to 18 and Table 1 below. For all plants tested, cultivation in 2-aminopimelic acid-containing SkyGel water-retaining medium resulted in decreased taproot length and increased lateral root density compared to cultivation in non-2-aminopimelic acid-containing SkyGel water-retaining medium. These results demonstrate that the fibrous root formation effect of 2-aminopimelic acid is effective not only in *Arabidopsis thaliana* but also in dicotyledonous plants of various other families.

**[Table 1]**

| | Increase rate of average lateral root density induced by 2-aminopimelic acid (%) |
|---|---|
| radish | 82 |
| broccoli | 169 |
| komatsuna | 95 |
| garden cress | 196 |
| arugula | 178 |
| mustard | 251 |
| sunflower | 87 |
| white sesame | 114 |
| red perilla | 329 |
| green perilla | 375 |
| red cabbage | 214 |
| alfalfa | 49 |
| mungbean | 166 |
| water morning glory | 30 |

2-aminopimelic acid significantly increased the number of lateral roots in sunflower and red cabbage. The average number of lateral roots when sunflower was cultivated in non-2-aminopimelic acid-containing or 2-aminopimelic acid-containing Sky Gel Medium was 50 and 61.5, respectively. The average number of lateral roots when red cabbage was cultivated in non-2-aminopimelic acid-containing or 2-aminopimelic acid-containing Sky Gel Medium was 14.4 and 27.6, respectively. These results suggest that root development can be promoted by 2-aminopimelic acid.

### Example 4: Effect of various concentrations of 2-aminopimelic acid on root system morphology.

To investigate the effect of various concentrations of 2-aminopimelic acid on root system morphology, following experiments were carried out.

According to the method described in Example 1, 2-aminopimelic acid-containing MS medium was prepared at a final concentration of 1 µM, 10 µM, or 100 µM.

*Arabidopsis thaliana* (Columbia-0) seeds were sterilized and sowed in 2-aminopimelic acid-containing MS medium in a square petri dish using the method described in Example 1. As a control, sterilized *Arabidopsis thaliana* seeds were sowed in non-2-aminopimelic acid-containing MS medium.

After sowing, the plant bodies were cultivated under photoperiodic conditions at 23°C with a light period of 16 hours and a dark period of 8 hours. After two weeks, the taproot length of the plant bodies was measured using Vernier calipers, and the number of lateral roots (number of primary lateral roots) of the plant bodies was also counted. The number of lateral roots was divided by the taproot length to calculate the lateral root density. The lateral root density of the plants cultivated in 2-aminopimelic acid-containing MS medium was calculated as the relative lateral root density, with the lateral root density of the plants cultivated in 2-aminopimelic acid-containing MS medium taken as 100%.

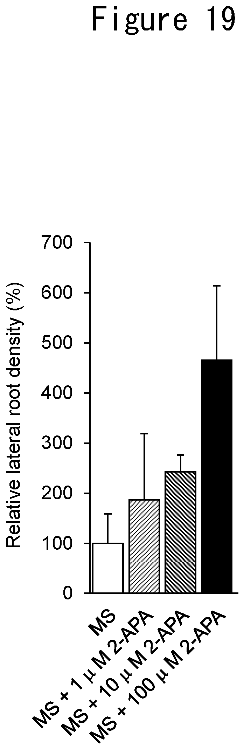

The results are shown in Figure 19. Lateral root density increased depending on the concentration of 2-aminopimelic acid.

### <Example 5: Effect of 2-aminopimelic acid in hydroponics>

To confirm the effect of 2-aminopimelic acid in hydroponics, following experiments were performed.

Using the method described in Example 3, non-2-aminopimelic acid-containing SkyGel water-retaining medium and 100 µM 2-aminopimelic acid-containing SkyGel water-retaining medium were prepared.

Komatsuna seeds (Nakahara Seed Co., Ltd.) were sowed in non-2-aminopimelic acid-containing SkyGel water-retaining medium or 100 µM 2-aminopimelic acid-containing SkyGel water-retaining medium, and then cultivated under photoperiodic conditions of 23°C with a light period of 16 hours and a dark period of 8 hours for approximately one week.

The resulting seedlings were transferred to a hydroponics kit (Home Hyponica Pukupuku II, Kyowa Co., Ltd.) and cultivated in a hydroponics environment. The hydroponic solution used was a 0.5 g/L Hyponex solution (Hyponex Japan Co., Ltd.) or a 0.5 g/L Hyponex solution with DL-2-aminopimelic acid (CAS RN: 627-76-9, Tokyo Chemical Industry Co., Ltd.) dissolved therein to a final concentration of 100 µM. Approximately one month after the start of cultivation, the fresh weight of the aerial part of the plant body and the fresh weight of the root organ were measured using an electronic balance (Shimadzu).

Figure 20 shows the root system morphology of komatsuna 12 and 24 days after sowing. Compared to komatsuna cultivated in the absence of 2-aminopimelic acid, komatsuna cultivated in the presence of 2-aminopimelic acid had shorter taproots, increased lateral root density, and trivialized rhizosphere. The average taproot length of komatsuna cultivated in the absence or presence of 2-aminopimelic acid 12 days after sowing was 7.58 cm and 3.29 cm, respectively. The average lateral root density of komatsuna cultivated in the absence or presence of 2-aminopimelic acid 12 days after sowing was 3.12 roots/cm and 6.09 roots/cm, respectively.

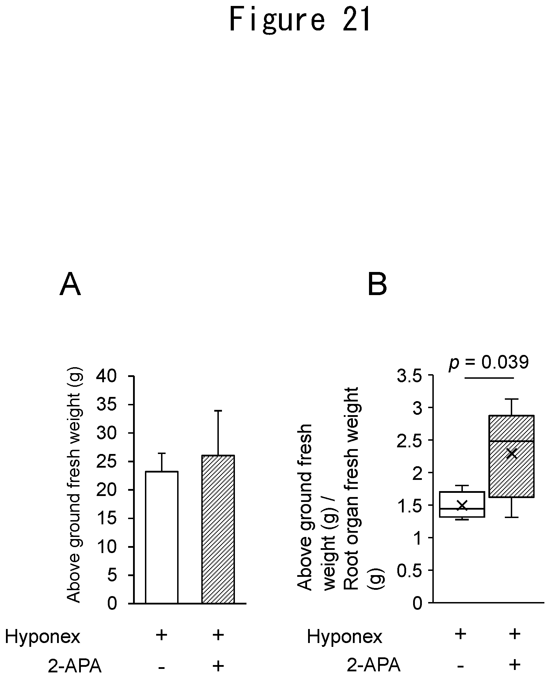

Figure 21 shows the results of measuring the fresh weight of the aerial parts of the plant body and the fresh weight of the root organ. Compared to komatsuna cultivated in the absence of 2-aminopimelic acid, komatsuna cultivated in the presence of 2-aminopimelic acid had a decreased root organ fresh weight, but a slight increase in the fresh weight of the aerial parts. A two-tailed Student's t-test showed a significant increase in the fresh weight of the aerial parts/root organ fresh weight. The increase in aerial part growth despite a decrease in the total root mass is thought to be due to an increase in nutrient absorption efficiency due to changes in root system morphology.

The trivialization of the rhizosphere and the reduction in the total amount of roots lead to a reduction in the amount of soil or water required for cultivation. Therefore, the above results show that cultivation in the presence of 2-aminopimelic acid can reduce the amount of soil or water used for cultivation without negative effects on the growth of the aerial part.

### <Example 6: Effect of L-2-aminopimelic acid on root system morphology>

In order to compare the effect of DL-2-aminopimelic acid and L-2-aminopimelic acid on root system morphology, following experiments were performed.

100 µM DL-2-aminopimelic acid-containing MS medium and non-2-aminopimelic acid-containing MS medium were prepared as described in Example 1. 100 µM L-2-aminopimelic acid-containing MS medium was also prepared by the same method, except that L-2-aminopimelic acid was used instead of DL-2-aminopimelic acid.

Surface-sterilized seeds of *Arabidopsis thaliana* Columbia-0 (Col-0) were sowed in DL-2-aminopimelic acid-containing MS medium, L-2-aminopimelic acid-containing MS medium, or non-2-aminopimelic acid-containing MS medium, followed by low temperature treatment and cultivation for 2 weeks. Then, taproot length and lateral root density were measured. The sowing, low temperature treatment, cultivation method, and methods for measuring taproot length and lateral root density are as described in Example 1.

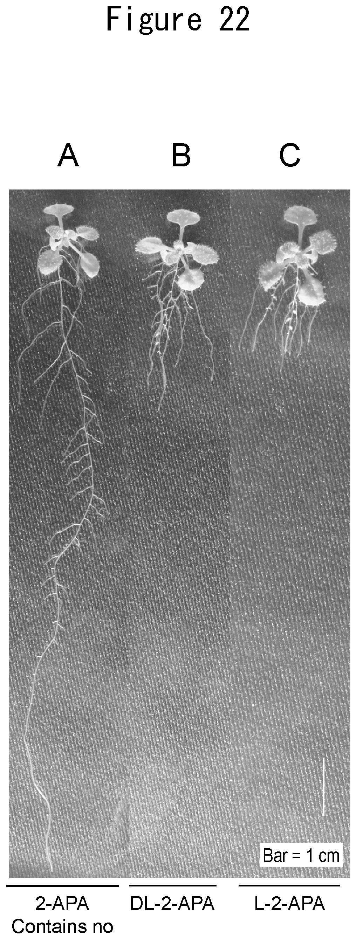

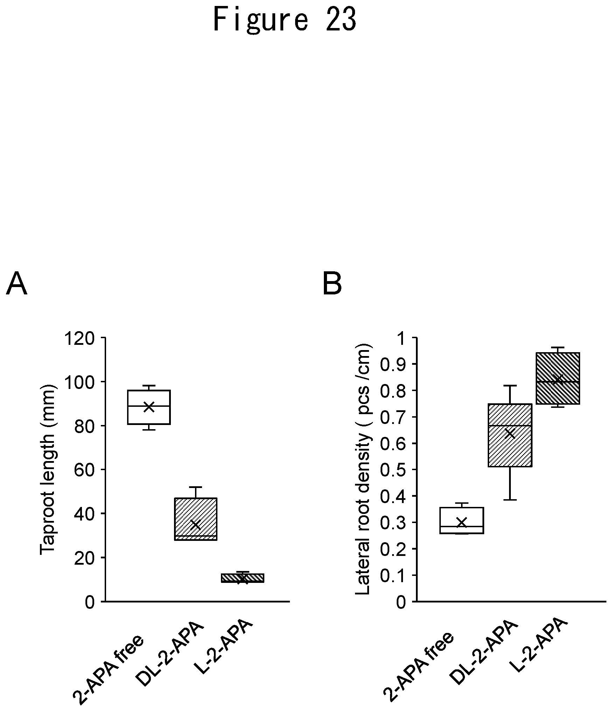

The results are shown in Figures 22 and 23. Both DL-2-aminopimelic acid and L-2-aminopimelic acid decreased taproot length and increased lateral root density, resulting in fibrous root formation in roots with a taproot-type root system. However, L-2-aminopimelic acid showed a stronger fibrous root formation effect than DL-2-aminopimelic acid.

All publications, patents, and patent applications cited in the description are incorporated in their entirety by reference.

## Claims

1. A root system morphology regulator of plants consisting of 2-aminopimelic acid or a salt or ester thereof.

2. The root system morphology regulator according to claim 1, wherein the controlled root system morphology is at least one selected from fibrous root formation, promotion of root hair growth, and rooting promotion.

3. The root system morphology regulator according to claim 1 or 2, wherein the plant is dicotyledonous plants.

4. A composition comprising the root system morphology regulator according to claim 1.

5. A method for controlling root system morphology of plants, comprising
a process of cultivating plants with a medium comprising 2-aminopimelic acid or a salt or ester thereof.

6. The method for controlling root system morphology of plants according to claim 5, wherein the controlled root system morphology is at least one selected from fibrous root formation, promotion of root hair growth, and rooting promotion.

7. A method for producing a plant, comprising
a process of cultivating a plant with a medium comprising 2-aminopimelic acid, or a salt or an ester thereof.

8. The method according to any one of claims 5 to 7, wherein the medium is a liquid medium.
